# EUROPEAN PATENT APPLICATION

(11) **EP 1 609 443 A1**
(43) Date of publication of application: **28.12.2005**
(21) Application number: 04724746.5
(22) Date of filing: 31.03.2004
(51) Int. Cl.: A61F 2/28

(54) **MATERIAL FOR REPAIRING BIOLOGICAL TISSUES AND PROCESS FOR PRODUCING THE SAME**

(30) Priority: 01.04.2003 JP 2003098031; 01.04.2003 JP 2003098032
(71) Applicant: Olympus Corporation, Tokyo 151-0072 (JP)
(72) Inventor: HAKAMATSUKA, Yasuharu, 1960033 (JP); TAKAMIYA, Yuji, Kobe-shi, Hyogo 6570068 (JP); SADAMORI, Katsuya, 1910022 (JP)
(74) Representative: von Hellfeld, Axel
(86) International application number: PCT/JP2004/004637
(87) International publication number: WO 2004/089254

(57) **Abstract**

A bone substitute (a material for repairing biological tissues) made from β-TCP as the raw material comprises a plate-type base member on the surface of which a plurality of grooves extending in a single direction are formed and separated from each other by partition wall members having an almost uniform thickness. This material sustains a sufficient binding force after application to a biological tissue and exhibits an excellent effect of forming a biological tissue. This bone substitute (material for repairing biological tissues) having a main body provided with a plurality of grooves extending in a single direction which are separated from each other by partition wall members having an almost uniform thickness, and a biodegradable viscous member which is filled into at least one of the penetrating holes of the main body, and contains an agonistic factor capable of increasing the cell activity, can provide agonistic factors of sufficient types and amounts to a biological tissue, and can control the sustained-release dose thereof.

## Description

### TECHNICAL FIELD

The present invention relates to a material for repairing biological tissues used for repairing a defect in biological tissues, and a process for producing the same.

### BACKGROUND ART

Recently, it has become possible to repair a defect in biological tissue such as bone caused by osteoncus extraction, trauma, or the like by regenerating the bone by filling a material for repairing biological tissues such as a bone substitute or the like. For such a bone substitute, hydroxyapatite (HAP) and tricalcium phosphate TCP) are known. However, from the viewpoint of leaving no foreign matter inside the body, a scaffold made of a porous calcium phosphate material, for example, such as β-TCP is used. If the β-TCP is left in contact with bone cells of a defect part of bone, so-called remodeling is performed in which osteoclasts eat the β-TCP and osteoblasts form a new bone. That is, the bone substitute filled in the defect part of the bone is replaced by autologous bone as time goes by (for example, refer to: Uemura and two others, "Tissue engineering in bone using biodegradable β-TCP porous material - a new material strengthened in vivo Osferion", Medical Asahi, The Asahi Shimbun Company, October 1, 2001, Vol. 30, No. 10, p. 38-41).

When the bone substitute is to be filled into a defect part of the bone, in order to increase the adhesiveness with the bone tissue, various inventions have been proposed in which: the surface to be in contact with the defect part of the bone is made uneven by plasma spraying (for example, refer to Japanese Patent No. 3166352); or a binding packing made from a nonwoven fabric is arranged on the surface in contact with the defect part of the bone (for example, refer to Japanese Unexamined Patent Application, First Publication No. 2003-19149).

On the other hand, according to the condition of the defect part of the bone, there is a case where the bone forming function is promoted by adding growth factors to the bone substitute in order to increase the activity of the cells surrounding the defect part of the bone.

As a method of adding the growth factors in this case, there is known a method wherein the bone substitute is provided with various sizes of holes which are filled with biodegradable viscous material such as collagens containing the growth factors, and the bone substitute is applied as a filling.

However, the binding force between the abovementioned conventional bone substitute, which is provided only with the uneven part or the binding packing, and the surrounding bone tissue is weak, so that the bone substitute and the bone tissues may become separated before the bone has grown.

Therefore, although such a structure has been employed, bone regeneration still takes a long time and it has been difficult to sufficiently demonstrate the bone regenerating function.

Moreover, in the abovementioned conventional bone substitute, although micropores are reliably in communication with each other inside of the porous body, there has been a problem in that straight penetrating holes are not provided and blood vessels can not be sufficiently formed. Moreover, there has been a problem in that growth factors of sufficient types and amounts can not be added, so that supply with a sustained-release property where they are leaked out into the biological tissue surrounding the defect as time goes by cannot be achieved, and therefore, the effect can not be maintained for a long time.

### DISCLOSURE OF INVENTION

In view of the above problems, an object of the present invention is to provide a material for repairing biological tissues which sustains a sufficient binding force after application to a biological tissue and promotes biological tissue formation.

Moreover, the present invention has an object of providing a material for repairing biological tissues which can provide agonistic factors of sufficient types and amounts to a biological tissue, and can control the sustained-release dose thereof.

The present invention employs the following solution in order to solve the above problems.

In the material for repairing biological tissues of the present invention, a plurality of grooves are formed in the surface of a plate-type base member.

Since a plurality of grooves are formed in the surface of the base member, the material for repairing biological tissues, can be more in contact with the biological tissues. As a result, places where the biological tissue formation is induced, are increased. Moreover, for example, if it is used as a bone substitute, it is filled so that its groove side is pressed against and inserted into the cancellous bone inside the bone, and its base plate side is in contact with the cortical bone being the outer layer, so that the adhesiveness with the cancellous bone is increased. As a result, even if a load is applied in a separation direction, the connection of the bone substitute with the biological tissues can be maintained, and the effect of forming a biological tissue can be maintained, enabling an increase in the healing speed.

Moreover, in the material for repairing biological tissues according to the present invention, it is preferable that the grooves be formed along almost the same direction.

According to the material for repairing biological tissues, since a plate-type base member can be readily formed into a curved shape along the direction where the grooves are extending, it can be provided in an optimum shape for defects in biological tissue.

A process for producing a material for repairing biological tissues of the present invention comprises: a step for supplying a raw material formed in a slurry form into a mold, and forming a compact in which a plurality of penetrating holes extending in a single direction are separated from each other by partition wall members having an almost uniform thickness; and a step for cutting and dividing the compact by a plane in parallel with the direction in which the penetrating holes are extending.

According to the process for producing the material for repairing biological tissues, by dividing the penetrating holes formed in the compact, grooves are formed by the partition wall members that divided the penetrating holes in the compact, and by pressing these grooves against the biological tissue, the adhesiveness thereof with the biological tissue can be improved. Moreover, since the compact is formed by supplying the raw material formed in a slurry form into the mold, the size and the shape of the penetrating holes which become the grooves, and the thickness of the partition wall member can be freely controlled, and the material for repairing biological tissues can be formed in an optimum shape and size considering the position to be repaired and the adhesiveness with the biological tissue.

The compact may have a honeycomb shape.

Moreover, another material for repairing biological tissues of the present invention comprises: a main body of a shape in which a plurality of penetrating holes extending in a single direction are formed separated from each other by partition wall members having an almost uniform thickness; and a biodegradable viscous member which is filled into at least one of the penetrating holes in the main body, and contains an agonistic factor capable of increasing cell activity.

In the main body of the material for repairing biological tissues, the size and shape of the penetrating holes can be freely controlled for molding. Moreover, since the biodegradable viscous member containing the agonistic factor is filled into the penetrating holes, then by controlling the position of the penetrating holes and the number thereof to be filled, the sequence of providing the agonistic factor into a defect, and the sustained-release dose can be controlled, so that the biological tissue formation can be effectively promoted.

The main body may be in a honeycomb shape.

### BRIEF DESCRIPTION OF DRAWINGS

FIG. 1 is a perspective view showing a material for repairing biological tissues according to a first embodiment of the present invention.
FIG. 2 shows a production flow of the material for repairing biological tissues according to the first embodiment of the present invention.
FIG. 3 is a perspective view showing a honeycomb compact according to the first embodiment of the present invention.
FIG. 4 is a perspective view showing a honeycomb mold used for production of the material for repairing biological tissues according to the first embodiment of the present invention.
FIG. 5 shows an example of use of the material for repairing biological tissues according to the first embodiment of the present invention.
FIG. 6 is a perspective view showing a material for repairing biological tissues according to a second embodiment of the present invention.
FIG. 7 shows a production flow of the material for repairing biological tissues according to the second embodiment of the present invention.
FIG. 8 is a perspective view showing a honeycomb compact according to the second embodiment of the present invention.
FIG. 9A to FIG. 9C show an intermediate product part way through production of the material for repairing biological tissues according to the second embodiment of the present invention.
FIG. 10 shows an example of use of the material for repairing biological tissues according to the second embodiment of the present invention.
FIG. 11 is a perspective view showing a general outline of a bone substitute main body according to a third embodiment of the present invention.
FIG. 12 is a front view showing the bone substitute according to the third embodiment of the present invention.
FIG. 13 shows a cross-section taken along the line II-II of FIG. 12.
FIG. 14 is a schematic diagram showing a bone forming function by the bone substitute according to the third embodiment of the present invention.
FIG. 15 is a front view showing another example of the bone substitute according to the third embodiment of the present invention.

### BEST MODE FOR CARRYING OUT THE INVENTION

### First Embodiment

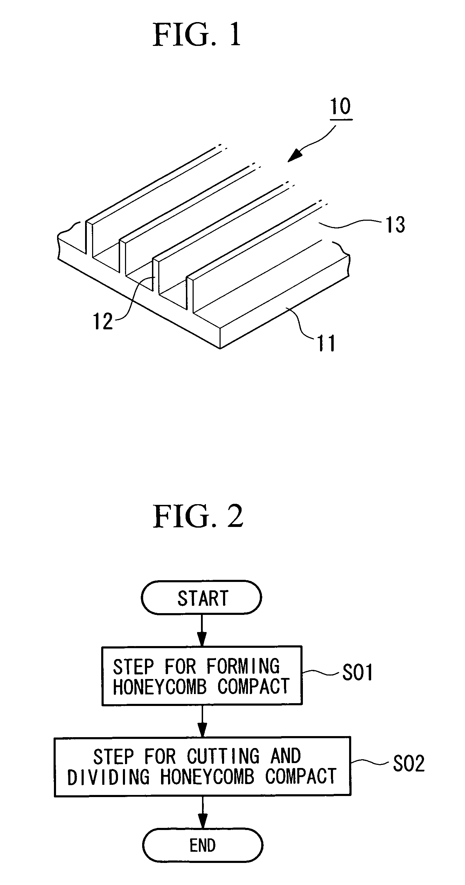

Hereunder is a description of a material for repairing biological tissues according to a first embodiment of the present invention, with reference to FIG. 1 to FIG. 4.

As shown in FIG. 1, a bone substitute (material for repairing biological tissues) 10 according to the present embodiment is made from β-TCP as the raw material, and comprises a plate-type base member 11 on the surface of which a plurality of grooves 12 extending in a single direction are formed and separated from each other by partition wall members 13 having an almost uniform thickness.

Next is a description of a process for producing the bone substitute 10.

As shown in FIG. 2, the production process for the bone substitute 10 according to the present embodiment comprises: a step (S01) for supplying a raw material formed in a slurry form into a honeycomb mold 14, and forming a honeycomb compact 15; and a step (S02) for cutting and dividing the honeycomb compact 15. Hereunder is a description of the respective steps.

In step (S01) for forming the honeycomb compact 15, firstly, for example by a method disclosed in Japanese Unexamined Patent Application, First Publication No. Hei 5-237178, an aqueous foamed slurry which has been mixed and foamed, is adjusted and mixed with granular compacts made from β-TCP so as to make a raw material which is formed in a slurry form.

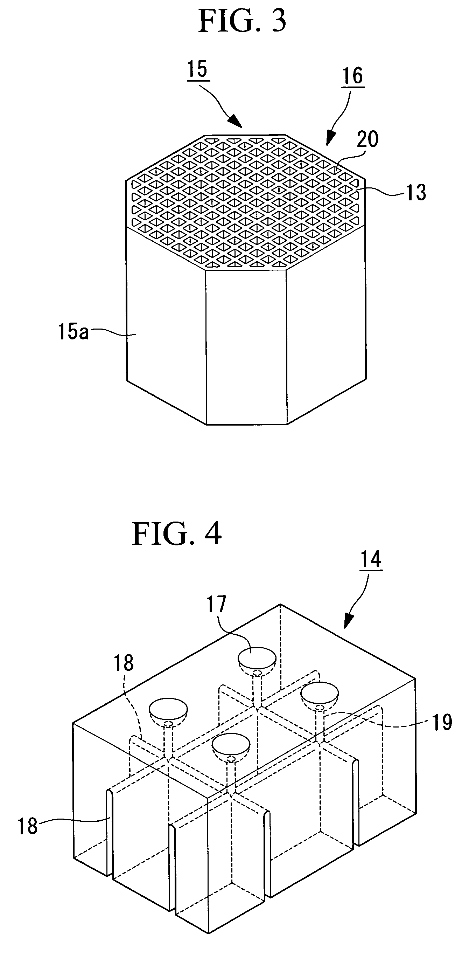

The raw material is supplied into the honeycomb mold 14 to make a honeycomb prototype 16 shown in FIG. 3.

As shown in FIG.4, in the top of the honeycomb mold 14 is formed raw material inlets 17 that have been formed in a funnel shape. Meanwhile, the inside at the bottom is formed with slits 18 extending respectively in parallel with the respective sides of the honeycomb mold 14. The bottom ends of the raw material inlets 17 and one end of the slits 19 are communicated through passages 19.

The width of the slits 18 and the intervals therebetween are previously determined considering the adhesiveness according to the shape and the size of the bone tissue to be repaired.

The obtained honeycomb prototype 16 is sintered after drying, for example in a method disclosed in Japanese Unexamined Patent Application, First Publication No. Hei 5-237178.

In this manner, the honeycomb compact 15 in which a plurality of penetrating holes 20 extending in a single direction are separated from each other by partition wall members 13, is obtained.

In the step (S02) for cutting and dividing the honeycomb compact 15, it is cut and divided by a plane (for example, the IV-IV position shown in FIG. 3) in parallel with the direction in which the penetrating holes 20 in the honeycomb compact 15 are extending.

In this case, the bone substitute 10 wherein the wall 15a constituting the external surface of the honeycomb compact 15 is serving as the base member 11, and the respective penetrating holes 20 are serving as grooves 12, is obtained.

Next is a description of the effect of the bone substitute 10 of the present embodiment having the above structure.

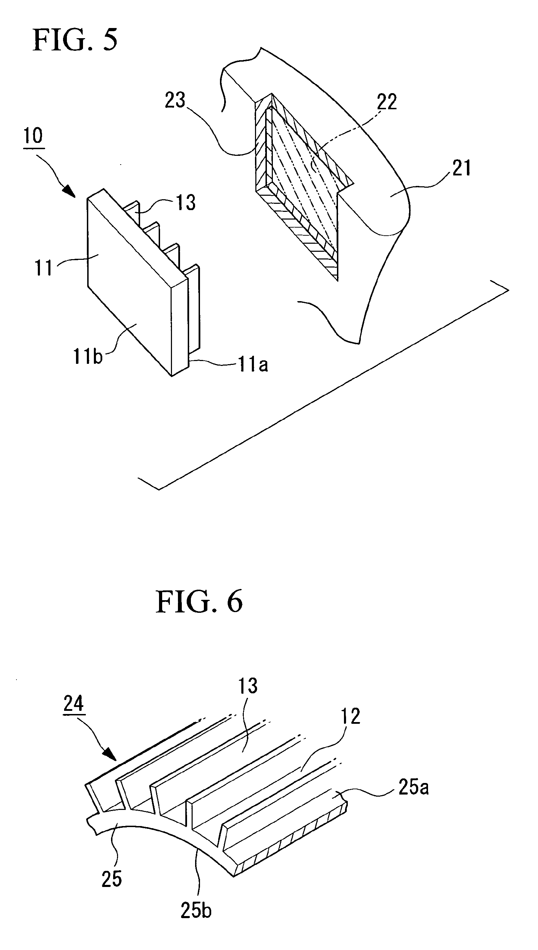

As shown in FIG. 5, the bone substitute 10 is filled into a defect in an ilium 21. At this time, the surface 11a formed with the grooves 12 is pressed against and inserted into the cancellous bone 22 in the ilium 21, to repair it so that the surface of the base member 11 without the grooves 12 is on the same surface with the cortical bone 23. In this case, since the partition wall members 13 is driven into the inside of the cancellous bone 22 like a wedge, the adhesiveness is mutually increased, and they are kept fixed even if the bone substitute 10 is loaded from the outside. At the same time, a new bone is formed using the bone substitute 10 as a scaffold.

Moreover, since the bone tissue and the bone substitute 10 are in contact over a large area including the surface of the partition wall members 13 as well as the surface of the base member 11, the bone is formed over a large area.

According to the bone substitute 10, since it is formed by honeycomb molding, the size and the shape of the penetrating holes 20 serving as the grooves 12 can be freely controlled, and it can be formed in an optimum shape and size considering the adhesiveness of the bone substitute to the bone to be repaired and its vicinity. Moreover, since the grooves 12 are formed, the bone substitute can be in contact with the cancellous bone 22 over a large area, and the bone formation can be promoted so as to increase the healing speed after the repair, more than in the conventional case.

### Second Embodiment

Next is a description of a material for repairing biological tissues according to a second embodiment of the present invention, with reference to FIG. 6 to FIG. 9. In the following description, the same reference symbols are used for components the same as those in the abovementioned embodiment, and description thereof is omitted.

The point where the second embodiment is different from the first embodiment is that in the first embodiment the material of the base member 11 of the bone substitute 10 is β-TCP, whereas in the second embodiment the material is a bioabsorbent material such as polylactic acid (PLA).

As shown in FIG. 6, the bone substitute 24 according to the present embodiment is made from PLA as the raw material, and comprises a curved base member 25 with a uniform thickness, on one surface 25a of which a plurality of grooves 12 extending in a single direction are formed and separated from each other by partition wall members 13 having an almost uniform thickness. On the other surface 25a of the base member 25 is applied β-TCP 26 together with surfactants.

Next is a description of a process for producing the bone substitute 24.

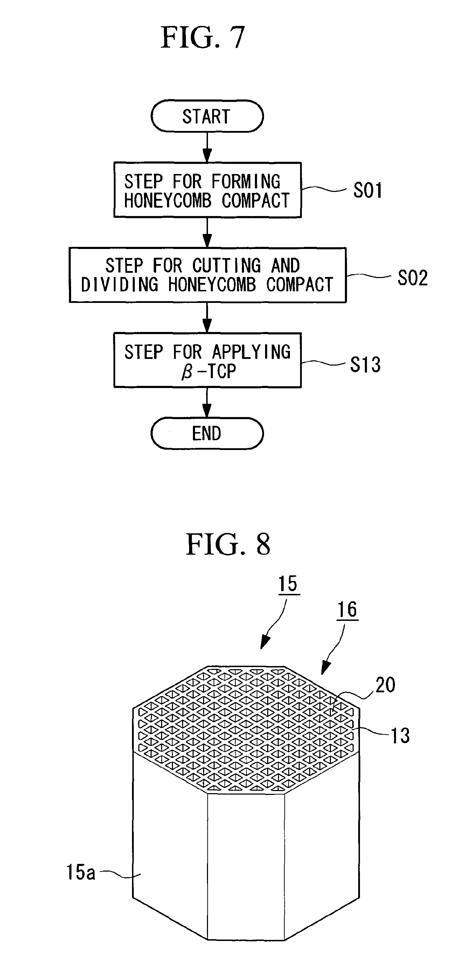

As shown in FIG. 7, similarly to the first embodiment, the production process for the bone substitute 24 according to the present embodiment comprises: a step (S01) for supplying a raw material formed in a slurry form into a honeycomb mold 14, and forming a honeycomb compact 15; and a step (S02) for cutting and dividing the honeycomb compact 15. The present embodiment further comprises a step (S13) for forming the divided body 27 obtained from the division in a curved shape, and then applying the β-TCP 26 onto the surface together with surfactants. Hereunder is a description of the respective steps.

In step (S01) for forming the honeycomb compact 15, firstly, the raw material of PLA that has been formed into a slurry form is made.

Similarly to the first embodiment, the raw material is supplied into the honeycomb mold 14 to make a honeycomb prototype 16.

The obtained honeycomb prototype 16 is then dried, and as shown in FIG. 8, the honeycomb compact 15 in which a plurality of penetrating holes 20 extending in a single direction are separated from each other by partition wall members 13, is obtained.

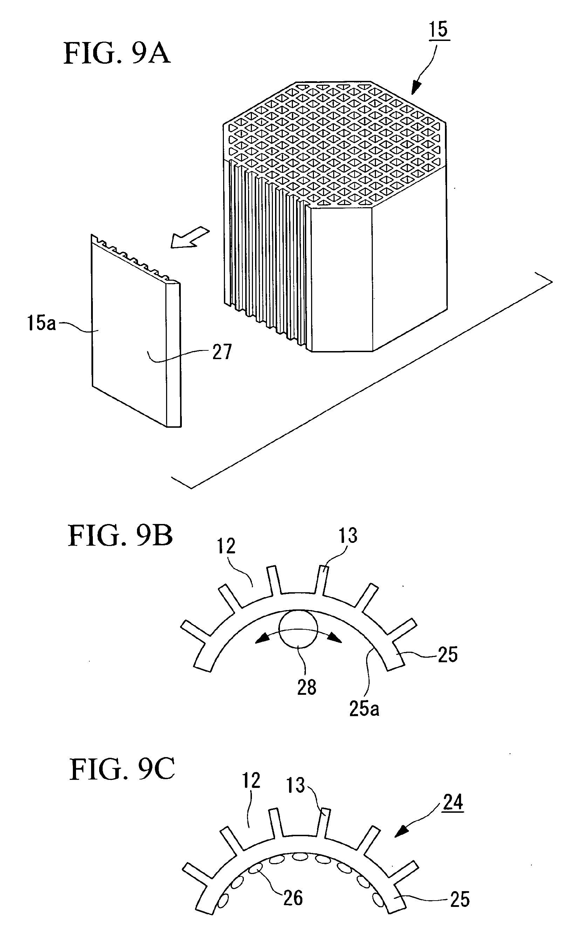

Next, the process proceeds to step (S02) for cutting and dividing the honeycomb compact 15. In this step, similarly to the first embodiment, as shown in FIG. 9A, it is cut so that the wall 15a of the external surface of the honeycomb compact 15 is serving as the base member 25, and the respective penetrating holes 20 are serving as grooves 12, so as to obtain the divided body 27.

In the step (S13) for applying the β-TCP 26, the base member 11 is heated, and then as shown in FIG. 9B, the divided body 27 is curved by a roller 28 extending in the direction of the grooves 12, so that the surface 25a of the base member 25 without the formed grooves 12 becomes the inner surface.

Then, as shown in FIG. 9C, on the surface 25a is applied surfactants and the β-TCP 26, so as to obtain the bone substitute 24.

Next is a description of the effect of the bone substitute 24 of the present embodiment having the above structure.

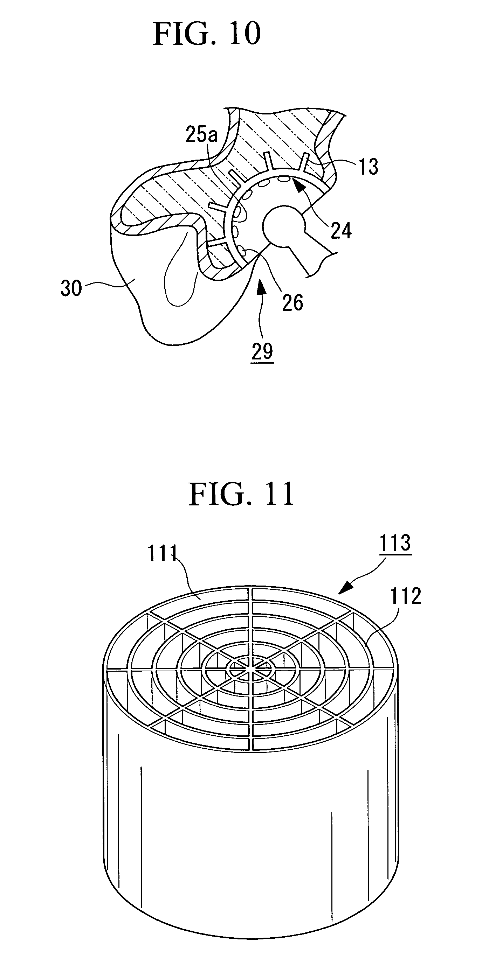

As shown in FIG. 10, the bone substitute 24 is inserted into a hip joint 29 and connected to a reamed pelvic acetabular bone 30. When fixing in this manner, the bone substitute 24 is squeezed, and the partition wall members 13 is driven into the inside of the cancellous bone in the acetabular bone 30 like a wedge, to be fixed. Therefore, even if the hip joint is loaded from various directions, it is not loosened.

Meanwhile, since the β-TCP 26 applied onto the surface 25a, and the bone cells being the defect part of the bone are in contact, so-called remodeling is performed in which osteoclasts eat the β-TCP 26 and osteoblasts form a new bone. Then, the bone substitute 24 is replaced by the autologous bone as time goes by.

According to the bone substitute 24, since it is formed by honeycomb molding, the size and the shape of the penetrating holes 20 serving as the grooves 12 can be freely controlled. Moreover, since after molding the base member 11 is curved and inserted, the adhesiveness of the bone substitute to the bone to be repaired and its vicinity can be further improved. Furthermore, since the β-TCP 26 is applied, then even if the bone substitute 24 is absorbed into the body, the bone regeneration is accelerated due to the bone forming function, and the healing speed after the repair can be increased more than for the conventional case.

The technical scope of the present invention is not limited by the abovementioned first and second embodiments, and various modifications can be made thereto without departing from the gist of the present invention.

For example, in the present embodiments, the base plate 24 is curved by heating. However, a honeycomb mold 14 which has been previously curved into a predetermined shape may be used to form the honeycomb compact 15 comprising a curved wall 15a.

Moreover, the shape of the penetrating holes 20 is not limited to the quadrangular shape, and may be a hexagonal shape, a triangular shape, or a circular shape, according to the part to be repaired. Moreover, the size of the respective penetrating holes are not necessarily uniform, and may be nonuniform.

The material of the honeycomb compact it is not necessarily β-TCP nor PLA, and provided it has an affinity with the biological tissue, it may be other bioabsorbent materials such as hydroxyapatite (HAP), or combinations thereof.

### Third Embodiment

Hereunder is a description of a material for repairing biological tissues according to a third embodiment of the present invention, with reference to FIG. 11 to FIG. 14.

A bone substitute (material for repairing biological tissues) 110 according to the present embodiment is made from β-TCP as the raw material and comprises: a honeycomb shaped main body 113 in which a plurality of penetrating holes 111 extending in a single direction are formed and separated from each other by partition wall members 112 having an almost uniform thickness; and collagens (biodegradable viscous member) 115 which are impregnated with growth factors (agonistic factor) 114 for increasing the cell activity, and filled into a plurality of penetrating holes 111.

As shown in FIG. 11, the partition wall members 112 separate the inside of the main body 113 in a concentric manner, forming a plurality of penetrating holes 111 that are formed in a sector shape.

The size of the penetrating holes 111 and the positions of the penetrating holes 111 that are filled with the collagens 115 are determined according to the sustained-release dose of the growth factors 114 which has impregnated into the collagens 115, and the amount of the collagens 115.

Next is a description of a process for producing the main body 113.

Firstly, for example, by a method disclosed in Japanese Unexamined Patent Application, First Publication No. Hei 5-237178, an aqueous foamed slurry which has been mixed and foamed, is adjusted and mixed with granular compacts made from β-TCP so as to make a raw material which is formed in a slurry form. By inserting the raw material into a honeycomb mold of a predetermined shape and size provided with slits, the honeycomb compact formed into a honeycomb shape is obtained, which is further sintered so as to obtain the main body 113.

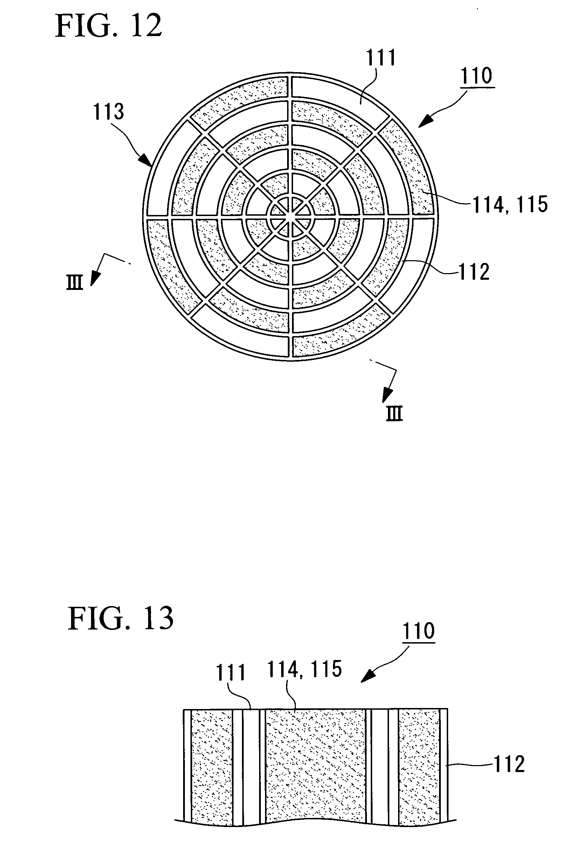

As shown in FIG. 12 and FIG. 13, the collagen 115 is filled in the penetrating holes 111 at predetermined positions. The collagen 115 is a kind of protein which functions as a structural material that supports the shape of the body and organs, and as an adhesive that adheres between cells, and performs biological activities such as cell proliferation, organ formation, promotion of healing a wound, and the like.

The collagen 115 is made into sol form at a temperature from 40°C to 60°C, then added with growth factors 114. This is then poured into the penetrating holes 111, and cooled to make into gel form, to thereby fill the penetrating holes 111.

The growth factor 114 is a cytokine such as PRP (platelet-rich plasma), and has a function of promoting bone regeneration, blood vessel formation, and the like.

Next is a description of the effect of the bone substitute 110 of the third embodiment having the above structure.

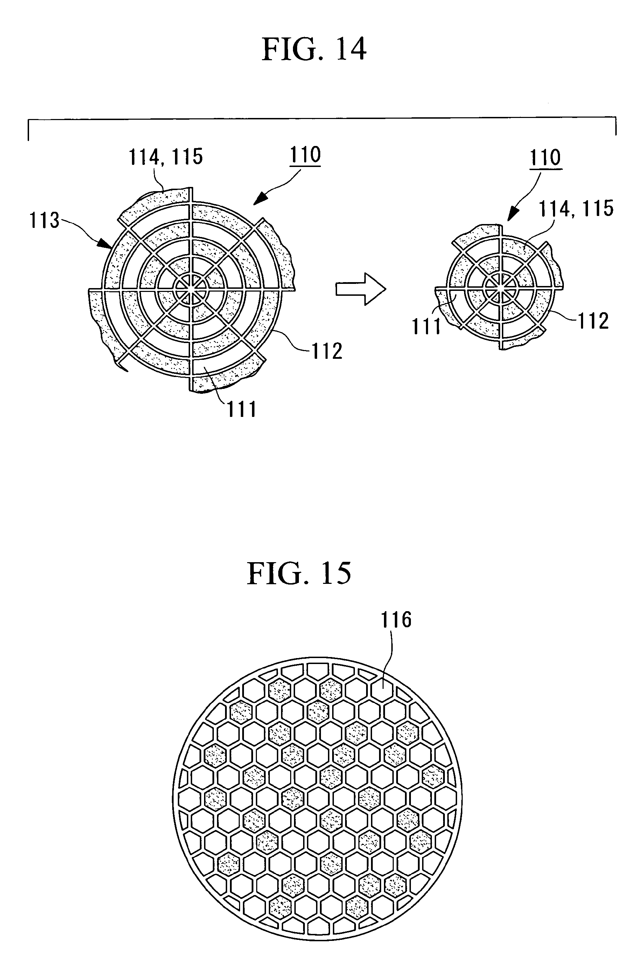

When the bone substitute 110 is grafted into a defect part of the bone, the bone regeneration is started from the periphery of the main body 113 which is in contact with the bone tissue. In this case, as shown in FIG. 14, the growth factors 114 are gradually released to the outside from the collagens 115 that have been filled in the periphery of the main body 113. Then, the surrounding osteoblasts are activated and the β-TCP constituting the main body 113 is absorbed from the periphery of the main body 113 so as to form bone.

Even if such absorption and bone formation progresses, since the penetrating holes 111 arranged in the center of the main body 113 are also impregnated with growth factor 114, then the function of the growth factors continues, and bone regeneration is performed, until the whole main body 113 is absorbed.

According to the bone substitute 110, since it is also impregnated with growth factors 114, then compared to the case where only β-TCP carriers are grafted for the repair, the activity of the surrounding tissue cells can be increased, and bone formation can be promoted.

Moreover, since it is formed into a honeycomb shape, the positions and the number of the penetrating holes 111 to be filled with the collagen 115 including the growth factor 114 can be determined, considering optimum sustained-release.

Furthermore, since the size and the shape of the penetrating holes can be freely controlled when making the honeycomb shape, an optimum shape can be provided for the condition of the affected part to be filled with the bone substitute 110.

The technical scope of the present invention is not limited by the abovementioned embodiments, and various modifications can be made without departing from the gist of the present invention.

For example, the shape of the penetrating holes is not limited to a sector shape, and may be a polygonal shape such as with the hexagonal penetrating holes 116 shown in FIG. 15, or a combination thereof. Moreover, the biodegradable viscous member to be filled into the penetrating holes is not limited to collagens, and may be proteins such as gelatin that is produced by extraction from collagens or other biodegradable viscous members. Furthermore, the arrangement and the size of the penetrating holes may be uniform or nonuniform.

For the material of the main body 113, it is not necessarily β-TCP, and provided it has an affinity with the biological tissue, it may be any material such as calcium phosphate ceramics, polylactic acid, or combinations thereof. Moreover, the agonistic factor is not limited to PRP, and may be any as long as it is required for the regeneration of biological tissues. Bone forming factors such as BMP (Bone Morphogenetic Protein), FGF (Fibroblast Growth Factor), TGF-β (Transforming Growth Factor-β), IGF (Insulin-like Growth Factor), PDGF (Platelet-Derived Growth Factor), VEGF (Vascular Endothelial cell Growth Factor), and the like can be filled solely or in combinations thereof.

### INDUSTRIAL APPLICABILITY

The following effects are demonstrated in the above described present invention.

According to the material for repairing biological tissues of the present invention wherein a plurality of grooves are formed in the surface of the plate-type base member, there is a sufficient binding force after application to a biological tissue, and the effect of forming a biological tissue can be promoted, so as to increase the healing speed of a defect in the biological tissue.

Using the material for repairing biological tissues according to the present invention, which comprises a honeycomb shaped main body in which a plurality of penetrating holes extending in a single direction are formed and separated from each other by partition wall members having an almost uniform thickness and a biodegradable viscous member which is filled into at least one of the penetrating holes in the main body and contains an agonistic factor capable of increasing the cell activity, agonistic factors of sufficient types and amounts can be supplied to a biological tissue, and the sustained-release dose thereof can be controlled, so that regeneration of the biological tissue can be performed under optimum conditions.

## Claims

1. A material for repairing biological tissues, wherein a plurality of grooves are formed in the surface of a plate-type base member.

2. A material for repairing biological tissues according to claim 1, wherein said grooves are formed along almost the same direction.

3. A process for producing a material for repairing biological tissues comprising:
a step for supplying a raw material formed in a slurry form into a mold, and forming a compact in which a plurality of penetrating holes extending in a single direction are separated from each other by partition wall members having an almost uniform thickness; and
a step for cutting and dividing said compact by a plane in parallel with the direction in which said penetrating holes are extending.

4. A process for producing a material for repairing biological tissues according to claim 3, wherein said compact is formed into a honeycomb shape.

5. A material for repairing biological tissues comprising:
a main body of a shape in which a plurality of penetrating holes extending in a single direction are formed separated from each other by partition wall members having an almost uniform thickness; and
a biodegradable viscous member which is filled into at least one of the penetrating holes in said main body, and contains an agonistic factor capable of increasing cell activity.

6. A material for repairing biological tissues according to claim 5, wherein said main body has a honeycomb shape.
